(19) **Europäisches Patentamt / European Patent Office / Office européen des brevets**

(11) **EP 4 635 556 A1**

(12) **EUROPEAN PATENT APPLICATION**
published in accordance with Art. 153(4) EPC

(43) Date of publication:
**22.10.2025 Bulletin 2025/43**

(21) Application number: **23905899.3**

(22) Date of filing: **18.12.2023**

(51) International Patent Classification (IPC):
***A61N 1/36*** (2006.01)

(52) Cooperative Patent Classification (CPC):
**A61N 1/05; A61N 1/36; G16H 20/40**

(86) International application number:
**PCT/CN2023/139589**

(87) International publication number:
**WO 2024/131736 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 CN 202211669107**

(71) Applicant: **Hangzhou Nuowei Medical Technology Co., Ltd**
**Hangzhou, Zhejiang 311100 (CN)**

(72) Inventors:
 • **CAO, Peng**
  **Hangzhou, Zhejiang 311100 (CN)**
 • **LIN, Ting**
  **Hangzhou, Zhejiang 311100 (CN)**
 • **QU, Xiao**
  **Hangzhou, Zhejiang 311100 (CN)**

(74) Representative: **Metida**
**Gyneju str. 16**
**01109 Vilnius (LT)**

(54) **SYSTEM AND DEVICE FOR NEURAL STIMULATION AND COMPUTER-READABLE STORAGE MEDIUM**

(57)    This disclosure provides a system for neural stimulation, a device for neural stimulation, and a computer-readable storage medium. The system includes a neural stimulator, the neural stimulator includes a stimulation module, configured to: determine, based on a received local field potential signal, a first energy value of a frequency band of interest in the local field potential signal; determine a threshold range in which the first energy value is; and output, according to a stimulation protocol corresponding to the threshold range, a corresponding stimulation signal. According to technical solution of the embodiments of this disclosure, a closed-loop neural stimulation mode can be achieved.

— 100

neural stimulator 110

stimulation module 111

FIG. 1

EP 4 635 556 A1

**Description**

**TECHNICAL FIELD**

This disclosure relates to the field of medical device technologies, in particular to a system for neural stimulation, a device for neural stimulation, and a computer-readable storage medium.

**BACKGROUND**

In traditional deep brain stimulation (DBS) systems for treating functional disorders such as Parkinson's disease, open-loop electrical stimulation therapy is commonly used. This open-loop stimulation therapy employs a fixed, continuous stimulation output manner, which cannot provide real-time precise modulation based on the patient's clinical symptoms or changes in disease conditions. Physicians typically adjust stimulation therapy parameters based on the patient's clinical symptoms and their own medical experience. As a result, when the patients' clinical symptoms or disease conditions change, they still need to be hospitalized for parameter adjustments by clinicians.

In view of this, there is an urgent need to provide a stimulation system that can adjust and control the stimulation protocol in a closed-loop manner.

**SUMMARY**

In order to at least address one or more of the above mentioned technical problems, this disclosure provides in multiple aspects a system for neural stimulation, a device for neural stimulation, and a computer-readable storage medium.

In a first aspect, this disclosure provides a system for neural stimulation, including a neural stimulator, where the neural stimulator includes a stimulation module, configured to: determine, based on a received local field potential signal, a first energy value of a frequency band of interest in the local field potential signal; determine a threshold range in which the first energy value is; and output, according to a stimulation protocol corresponding to the threshold range, a corresponding stimulation signal.

In some embodiments, the stimulation module is further configured to: before determining the threshold range in which the first energy value is, determine, based on a local field potential signal of a patient in at least one first state, a second energy value of the frequency band of interest in the local field potential signal in each of the at least one first state; where the system further includes a control terminal, configured to: determine, according to at least one received second energy value corresponding to at least one first state, a plurality of threshold ranges; and determine, based on the plurality of threshold ranges, respective stimulation protocols.

In some embodiments, the control terminal is further configured to: sort, in response to receiving second energy values in the at least one second energy value, the second energy values according to sizes of the second energy values; and determine, by using the sorted second energy values as endpoint values, the plurality of threshold ranges.

In some embodiments, the at least one first state includes at least one of following: unmedicated and the neural stimulator being not turned on for treatment; unmedicated and the neural stimulator being turned on for treatment; medicated and the neural stimulator being not turned on for treatment; or medicated and the neural stimulator being turned on for treatment.

In some embodiments, the stimulation module is further configured to: before determining the first energy value, determine, based on local field potential signals of the patient in a plurality of second states, respective third energy values of a plurality of frequency bands in the local field potential signals in the second states; and the control terminal is further configured to: determine, based on a change amount between the third energy values of the frequency bands in the plurality of second states, the frequency band of interest in the plurality of frequency bands.

In some embodiments, the plurality of second states includes at least one of following: unmedicated with the neural stimulator being not turned on for treatment and medicated with the neural stimulator being not turned on for treatment; or medicated with the neural stimulator being not turned on for treatment and medicated with the neural stimulator being turned on for treatment.

In some embodiments, the stimulation module is further configured to: determine the first energy value based on following formulas: $S_x(k) = \frac{1}{N}|X[k]|^2$; $X[k] = \sum_{n=0}^{N-1} x[n] \times e^{-jkn\omega} = \sum_{n=0}^{N-1} x[n] \times e^{-j2\pi kn/N}$; where, $S_x$ (k) represents an energy value, $x[n]$ represents a finite length sequence of length N in the frequency band of interest, $X[k]$ is a discrete Fourier transform pair of the $x[n]$ sequence, $0 \leq k \leq N-1$, N represents the sequence length, a complex exponential signal $e^{jwt} = cos(wt) - jsin(wt)$, an angular frequency of a periodic signal $\omega=2\pi/N$, e represents a natural constant, and j represents an imaginary unit.

In some embodiments, the system further includes: an electrode, configured to collect the local field potential signal of the patient's brain region and output a stimulation electrical pulse according to the received stimulation signal; and the neural stimulator further includes a collecting module, configured to receive the local field potential signal collected by the electrode and send the local field potential signal to the stimulation module.

In some embodiments, the neural stimulator further includes: a first communication module, configured to send the second energy value; and a microcontroller unit, configured to control the stimulation module and the first communication module. The control terminal includes: a second communication module, connected to the first communication module and configured to receive the second energy value, and to send the threshold ranges and corresponding stimulation protocols.

In a second aspect, this disclosure provides a device for neural stimulation, including: a processor, configured to execute program instructions, and a memory having program instructions stored thereon, where the program instructions, when loaded and executed by the processor, cause the device to perform following operations: determining, based on a received local field potential signal, a first energy value of a frequency band of interest in the local field potential signal; determining a threshold range in which the first energy value is; and outputting, according to a stimulation protocol corresponding to the threshold range, a corresponding stimulation signal.

In some embodiments, the program instructions, when loaded and executed by the processor, further cause the device to perform following operations: before determining the threshold range in which the first energy value is, determining, based on a local field potential signal of a patient in at least one first state, a second energy value of the frequency band of interest in the local field potential signal in each of the at least one first state; determining, according to at least one second energy value corresponding to at least one first state, a plurality of threshold ranges; and determining, based on the plurality of threshold ranges, respective stimulation protocols.

In some embodiments, the program instructions, when loaded and executed by the processor, further cause the device to perform following operations in determining the plurality of threshold ranges: sorting, in response to receiving second energy values in the at least one second energy value, the second energy values according to sizes of the second energy values; and determining, by using the sorted second energy values as endpoint values, the plurality of threshold ranges.

In some embodiments, the at least one first state includes at least one of following: unmedicated and the neural stimulator being not turned on for treatment; unmedicated and the neural stimulator being turned on for treatment; medicated and the neural stimulator being not turned on for treatment; or medicated and the neural stimulator being turned on for treatment.

In some embodiments, the program instructions, when loaded and executed by the processor, further cause the device to perform following operations: before determining the first energy value, determining, based on local field potential signals of the patient in a plurality of second states, third energy values of a plurality of frequency bands in the local field potential signals in the second states; and determining, based on a change amount between the third energy values of the frequency bands in the plurality of second states, the frequency band of interest in the plurality of frequency bands.

In some embodiments, the plurality of second states includes at least one of following: unmedicated with the neural stimulator being not turned on for treatment and medicated with the neural stimulator being not turned on for treatment; or medicated with the neural stimulator being not turned on for treatment and medicated with the neural stimulator being turned on for treatment.

In some embodiments, the program instructions, when loaded and executed by the processor, further cause the device to perform following operations in determining the first energy value: determining the first energy value based on following formulas: $S_x(k) = \frac{1}{N}|X[k]|^2$; $X[k] = \sum_{n=0}^{N-1} x[n] \times e^{-jkn\omega} = \sum_{n=0}^{N-1} x[n] \times e^{-j2\pi kn/N}$ ; where, $S_x(k)$ represents the energy value, $x[n]$ represents a finite length sequence of length N in the frequency band of interest, $X[k]$ is a discrete Fourier transform pair of the $x[n]$ sequence, $0 \le k \le N-1$, N represents the sequence length, a complex exponential signal $e^{jwt} = cos(wt) - jsin(wt)$, an angular frequency of a periodic signal $\omega=2\pi/N$, e represents a natural constant, and j represents an imaginary unit.

In a third aspect, this disclosure provides a computer readable storage medium having computer-readable instructions stored thereon, the computer-readable instructions implementing, when executed by one or more processors, the operations performed by any device as described in the second aspect of this disclosure.

Through the technical solution for neural stimulation, the system in the embodiments of this disclosure can determine the first energy value of the frequency band of interest in the local field potential signal through the stimulation module, and output the corresponding stimulation signal according to the threshold range in which the first energy value is, thereby achieving a closed-loop neural stimulation mode, that is, being able to timely adjust the stimulation protocol according to the change of the energy value of the local field potential signal collected in real time. Furthermore, in some embodiments, the third energy values of multiple frequency bands in the local field potential signal under multiple second states are determined by the stimulation module, so as to determine the frequency band of interest according to the change amount in the third energy values of the frequency bands in different second states, and determine the frequency band of interest with a higher correlation with the disease, thereby to improve the accuracy and effects of the stimulation protocol and avoid blind overstimulation.

## BRIEF DESCRIPTION OF THE DRAWINGS

The above and other objects, features and advantages of the exemplary embodiments of this disclosure will become easier to understand by reading the detailed description below with reference to the accompanying drawings. In the accompanying drawings, several embodiments of this disclosure are shown in an illustrative and non-limiting manner, and the same or corresponding reference numerals represent the same or corresponding elements, where:

FIG. 1 shows a schematic block diagram of a system for neural stimulation according to the embodiments of this disclosure;
FIG. 2 is a schematic flow chart showing the output of stimulation signals by the stimulation module according to the embodiments of this disclosure;
FIG. 3 shows a graph of a first energy value determined in real time according to the embodiments of this disclosure;
FIG. 4 is a schematic diagram showing adjusting a stimulation protocol according to a change in the first energy value according to the embodiments of this disclosure;
FIG. 5 shows a schematic block diagram of a system including a control terminal according to the embodiments of this disclosure;
FIG. 6 shows a schematic block diagram of a system including an electrode according to the embodiments of this disclosure;
FIG. 7 shows a line graph of the third energy values of multiple frequency bands in various second states according to the embodiments of this disclosure; and
FIG. 8 shows a schematic block diagram of a device for neural stimulation according to the embodiments of this disclosure.

## DETAILED DESCRIPTION

The technical solutions in the embodiments of this disclosure will be described hereinafter clearly and completely with reference to the drawings of the embodiments of this disclosure. Apparently, the following embodiments merely relate to a part of, rather than all of, the embodiments of this disclosure, and based on these embodiments, a person of ordinary skill in the art may, without any creative effort, obtain other embodiments, which also fall within the scope of this disclosure.
As can be appreciated, the terms "comprises," "comprising," "includes," and "including," when used in this specification and the claims, specify the presence of stated features, integers, steps, operations, elements, and/or components thereof, but do not preclude the presence or addition of one or more other features, integers, steps, operations, elements, components, and/or groups thereof.
As can be further appreciated, the terminology used herein is for the purpose of describing particular embodiments and is not intended to be limiting. As used herein, the singular forms, "a," "an," and "the" are intended to include the plural forms as well, unless the context clearly indicates otherwise. In addition, the expression "and/or" is used to indicate the existence of all or any one of one or more of listed items, or combinations thereof.
As used in this specification and the claims, the term "if" may, depending on the context, be interpreted as "when" or "upon" or "in response to determining" or "in response to detecting." Correspondingly, the phrase "if it is determined" or "if [a stated condition or event] is detected" may, depending on the context, be interpreted to mean "upon determining" or "in response to determining" or "upon detecting [the stated condition or event]" or "in response to detecting [the stated condition or event]."
The specific implementations of this disclosure will be described in detail below with reference to the accompanying drawings.

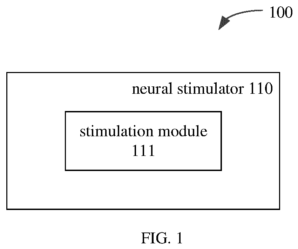

FIG. 1 shows a schematic block diagram of a system for neural stimulation according to the embodiments of this disclosure. As shown in FIG. 1, the system 100 for neural stimulation includes a neural stimulator 110, and the neural stimulator 110 includes a stimulation module 111, configured to: determine, based on a received local field potential signal, a first energy value of a frequency band of interest in the local field potential signal; determine a threshold range in which the first energy value is; and output, according to a stimulation protocol corresponding to the threshold range, a corresponding stimulation signal.
The neural stimulator 110 may be implanted in a patient's body. In some embodiments, the neural stimulator 110 may be implanted in the patient's skull. The local field potential (LFP) signal is an instantaneous electrical signal generated by the superposition and synchronization of cellular electrical activities in neural tissues or other tissues. In some embodiments, the received local field potential signal may be filtered, denoised, etc., so as to process the local field potential signal into a plurality of frequency bands, and the frequency band of interest may be one or more of the plurality of frequency bands. In some embodiments, the plurality of frequency bands may include, for example, Delta frequency band (0-3Hz), Theta frequency band (4Hz-7Hz), Alpha frequency band (8Hz-12Hz), Beta frequency band (13Hz-35Hz), Gamma frequency band (36Hz -200Hz), etc. The frequency band segmentation of the local field potential signal may not be limited to this, and may be divided into more or less as needed. In some embodiments, the frequency band of interest may be set as required, for example, the frequency band of interest may be a frequency band related to the disease to be treated.

The first energy value may be obtained through a frequency spectrum, where the frequency spectrum represents the relationship between the signal frequency and the energy. The first energy value may also be calculated in combination with the time-frequency, where the time-frequency represents the relationship between the time and the signal frequency. In some embodiments, the method for determining the first energy value may adopt such classical algorithms as direct methods (e.g., a periodogram method), indirect methods (e.g., an autocorrelation function method) or improved direct methods (e.g., the Barlett's method, Welch's method and Nuttall's method). In some embodiments, the determination of the first energy value may be implemented by using such algorithms as parametric model-based power spectrum calculation methods and non-parametric model-based power spectrum calculation methods. The parametric model-based power spectrum calculation methods may include methods based on AutoRegressive AR model, Moving Average MA model, AutoRegressive Moving Average ARMA model and the like. The non-parametric model-based power spectrum calculation methods may include such power spectrum estimation algorithms based on matrix eigendecomposition as power spectrum estimation based on Multiple Signal Classification MUSIC algorithm or power spectrum estimation algorithm based on eigenvector.

In other embodiments, the stimulation module 111 is further configured to: perform discrete Fourier transform on a time sequence signal of the frequency band of interest, and determine the first energy value according to a ratio of a square of a result of the discrete Fourier transform to a quantity of signal points of the time sequence signal. In specific, in some embodiments, the stimulation module 111 is further configured to: determine the first energy value based on following formulas:

$$S_x(k) = \frac{1}{N}|X[k]|^2$$

$$X[k] = \sum_{n=0}^{N-1} x[n] \times e^{-jkn\omega} = \sum_{n=0}^{N-1} x[n] \times e^{-j2\pi kn/N}$$

where, $S_x(k)$ represents the energy value, $x[n]$ represents a finite length sequence of length N in the frequency band of interest, $X[k]$ is a discrete Fourier transform pair of the $x[n]$ sequence, $0 \leq k \leq$ N-1, k may represent the frequency, N represents the sequence length (or the quantity of signal points), n represent a ordinal number of a signal point (i.e., the n-th signal point), a complex exponential signal $e^{jwt} = cos(wt) - jsin(wt)$, an angular frequency of a periodic signal $\omega = 2\pi/N$, e represents a natural constant, and j represents an imaginary unit.

After determining the first energy value of the frequency band of interest, the stimulation module 111 may determine the threshold range in which the first energy value is. In some embodiments, a plurality of threshold ranges may be pre-set, and the threshold range in which the first energy value is may be determined by determining whether the first energy value falls within any of the plurality of threshold ranges. The plurality of threshold ranges may correspond to different stimulation protocols respectively, so that after the stimulation module 111 determines the threshold range in which the first energy value is, it can directly output a corresponding stimulation signal according to the stimulation protocol corresponding to the threshold range. Through this configuration, the neural stimulator itself may adjust a more reasonable stimulation output in time according to the real-time changes in the energy value of the local field potential signal, without relying on a physician to make adjustments, thereby realizing closed-loop control of the stimulation therapy.

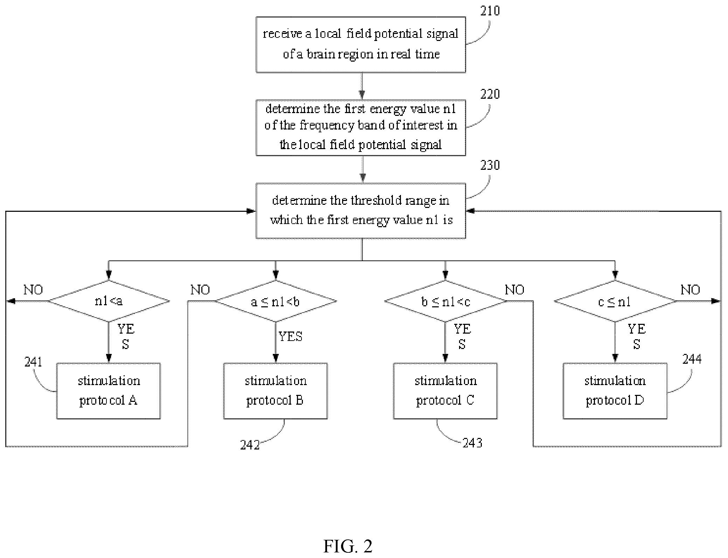

In some embodiments, the stimulation protocol may include a setting protocol for at least one stimulation parameter of an amplitude, a frequency, and a pulse width of a stimulation wave, and outputting the corresponding stimulation signal includes outputting at least one stimulation parameter of the corresponding amplitude, frequency, and pulse width, or outputting a stimulation wave formed by the stimulation parameter. In some embodiments, the stimulation module 111 may be implemented by hardware and/or software, for example, in the form of an integrated circuit. In order to facilitate understanding of the operations performed by the stimulation module 111, an exemplary description will be provided below in conjunction with FIG. 2.

FIG. 2 is a schematic flow chart showing the output of stimulation signals by the stimulation module according to the embodiments of this disclosure. As shown in FIG. 2, at step 210, the stimulation module may receive a local field potential signal of a brain region in real time. In some embodiments, the local field potential signal from the brain region may be obtained in real time by a brain electrode. Next, at step 220, the stimulation module may determine the first energy value n1 of the frequency band of interest in the real-time local field potential signal based on the local field potential signal received in real time. As can be appreciated, since the local field potential signal is obtained in real time, the first energy value n1 may be obtained in real time. The specific implementation of step 220 has been described above in detail in conjunction with FIG. 1, which will not be elaborated again herein.

Next, the process may proceed to step 230, where the threshold range in which the first energy value n1 is may be determined. Specifically, assuming that the preset plurality of threshold ranges includes: $(-\infty, a)$, $[a, b)$, $[b, c)$ and $[c, +\infty)$, where a, b, and c represent endpoint values of the respective threshold ranges and may increase sequentially. Each threshold range may correspond to at least one stimulation protocol. For example, as shown in FIG. 2, the threshold range $(-\infty, a)$ may correspond to stimulation protocol A, the threshold range $[a, b)$ may correspond to stimulation protocol B, the threshold range $[b, c)$ may correspond to stimulation protocol C, and the threshold range $[c, +\infty)$ may correspond to stimulation protocol D.

When the stimulation module performs step 230, the first energy value may be compared with a, b, and c respectively to determine the threshold range in which the first energy value n1 is. In response to the first energy value n1 being in the threshold range $(-\infty, a)$, i.e., $n1 < a$, step 241 may be executed to run the stimulation protocol A, and to output a corresponding stimulation signal according to the stimulation parameters set in the stimulation protocol A. In response to the first energy value n1 being in the threshold range $[a, b)$, i.e., $a \leq n1 < b$, step 242 may be executed to run the stimulation protocol B, and to output a corresponding stimulation signal according to the stimulation parameters set in the stimulation protocol B. In response to the first energy value n1 being in the threshold range $[b, c)$, i.e., $b \leq n1 < c$, step 243 may be executed to run the stimulation protocol C, and to output a corresponding stimulation signal according to the stimulation parameters set in the stimulation protocol C. In response to the first energy value n1 being in the threshold range $[c, +\infty)$, i.e., $n1 \geq c$, step 244 may be executed to run the stimulation protocol D, and to output a corresponding stimulation signal according to the stimulation parameters set in the stimulation protocol D.

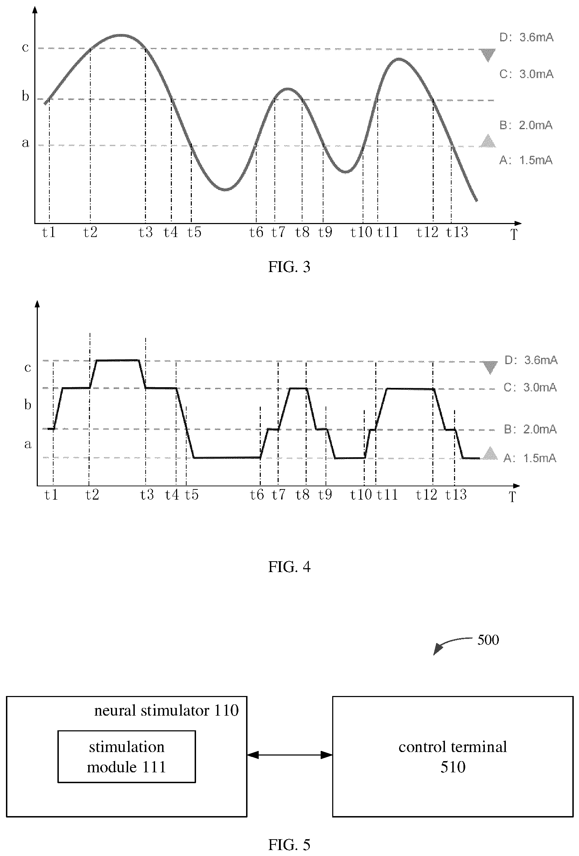

With such configuration, the stimulation module may automatically output more appropriate stimulation signals in real time based on the received local field potential signals in real time, rather than delivering fixed continuous stimulation until manual adjustment by a physician. Furthermore, to facilitate understanding of the specific process by which the stimulation module dynamically adjusts the stimulation protocol in real time according to the real-time determined threshold range of the first energy value, an exemplary description will be provided below with reference to FIG. 3 and FIG. 4.

FIG. 3 shows a graph of the first energy value determined in real time according to the embodiments of this disclosure. When the stimulation module determines the first energy value of the frequency band of interest in the received local field potential signal in real time, it is able to obtain such an energy graph as that shown in FIG. 3. As shown in FIG. 3 , the horizontal axis T in the graph represents time, the vertical axis represents energy value, a, b, and c (indicated by dashed lines in the figure) represent the endpoint values of respective threshold ranges, and t1, t2, t3, t4, t5, t6, t7, t8, t9, t10, t11, t12, and t13 (marked by dot-dash lines at their corresponding positions on the curve) represent different time points. In some embodiments, a may be set to, for example, 1.14 $\mu$Vp, b may be set to, for example, 1.41 $\mu$Vp, and c may be set to, for example, 1.67 $\mu$Vp.

As further illustrated in the figure, within the threshold range $(-\infty, a)$, the stimulation protocol A may be applied. Within the threshold range $[a, b)$, the stimulation protocol B may be applied. Within the threshold range $[b, c)$, the stimulation protocol C may be applied. Within the threshold range $[c, +\infty)$, the stimulation protocol D may be applied. In a specific implementation, different stimulation protocols may be configured by varying one stimulation parameter while keeping other stimulation parameters fixed. For example, the stimulation protocol A may include an output current of 1.5 mA (i.e., amplitude), the stimulation protocol B may include an output current of 2.0 mA, the stimulation protocol C may include an output current of 3.0 mA, and the stimulation protocol D may include an output current of 3.6 mA. In addition, the frequency in the stimulation protocols A, B, C, and D may all be set to 200 Hz, and the pulse width thereof may be set to 160 $\mu$s.

FIG. 4 is a schematic diagram showing adjusting a stimulation protocol according to a change in the first energy value according to the embodiments of this disclosure. Figure 4 is a schematic diagram illustrating the adjustment of the output stimulation protocol based on the energy curve shown in FIG. 3. As shown in FIG. 4, the horizontal axis (T) of the line graph represents time, while the vertical axis represents the stimulation current value outputted by the system according to the embodiments of this disclosure. A, B, C, and D (indicated by dashed lines in the figure) denote the stimulation current values set of the stimulation protocols, and a, b, and c represent the endpoint values of the respective threshold ranges.

The following may be observed with reference to FIG. 3 and FIG. 4. In the energy value curve shown in FIG. 3, before time point t1, the first energy value lies between a and b, corresponding to the stimulation protocol B. Therefore, as illustrated in the schematic diagram of performing the stimulation protocol in FIG. 4, up to time point t1, the stimulation current outputted by the system remains at the level of the stimulation protocol B. Furthermore, as shown in FIG. 3, from t1 to t2, the first energy value is between b and c, corresponding to the stimulation protocol C. Accordingly, as shown in FIG. 4, from t1 to t2, the stimulation current outputted by the system gradually increases to the level specified by the stimulation protocol C and then remains stable. After t2, due to changes in the first energy value, the corresponding stimulation protocol is changed, leading to a corresponding change in the output stimulation current. Similarly, the stimulation outputs at time points t3 to t13 and beyond vary in response to changes in the first energy value, which will not be elaborated again herein.

The system of the closed-loop control stimulation protocol according to the embodiment of this disclosure is described above with reference to FIG. 1 to FIG. 4. As can be appreciated, when the stimulation outputs are adjusted by the stimulation module according to the changes in the threshold range in which the first energy value is in the embodiment of the present application, it is able to automatically adjust the stimulation protocol in real time according to the real-time changes in the patient's condition, so as to form a continuous adaptive closed-loop stimulation adjustment, rather than a fixed stimulation output. As can be further appreciated, the above description is illustrative rather than limitative. For example, the quantity of threshold ranges may not be limited to four shown in FIG. 2, and may also be set to more or fewer in accordance with the practical needs. The endpoint values of the threshold ranges (such as a, b, c in FIG. 2 to FIG. 4) may be set in accordance with the practical needs. For another example, the quantity of stimulation protocols is not limited to four in the figures, and may also be set to more or fewer in accordance with the practical needs. The stimulation protocols are not limited to setting the stimulation current output, but may also be configured to adjust a stimulation voltage or other parameters in accordance with the practical needs. Furthermore, the system according to the embodiments of this disclosure is not limited to only including the neural stimulator, but may also include other devices. An exemplary description will be provided below with reference to FIG. 5.

FIG. 5 shows a schematic block diagram of a system including a control terminal according to the embodiments of this disclosure. The system 500 may include the neural stimulator 110 and a control terminal 510, the neural stimulator 110 may include the stimulation module 111, and the stimulation module 111 is further configured to: before determining the threshold range in which the first energy value is, determine, based on a local field potential signal of a patient in at least one first state, a second energy value of the frequency band of interest in the local field potential signal in each of the at least one first state. The control terminal, configured to: determine, according to at least one received second energy value corresponding to at least one first state, a plurality of threshold ranges; and determine, based on the plurality of threshold ranges, respective stimulation protocols.

In some embodiments, the first state may include unmedicated and the neural stimulator being not turned on for treatment, unmedicated and the neural stimulator being turned on for treatment, medicated and the neural stimulator being not turned on for treatment, medicated and the neural stimulator being turned on for treatment, and etc. In some application scenarios, at least one first state may include one first state, such as unmedicated and the neural stimulator is turned on for treatment. In other application scenarios, at least one first state may include multiple first states, such as unmedicated and the neural stimulator being not turned on for treatment, as well as medicated and the neural stimulator being turned on for treatment.

The stimulation module 111 may determine the second energy value of the frequency band of interest in the local field potential signal in each first state based on the local field potential signal in each first state, so as to obtain multiple threshold ranges determined according to at least one second energy value corresponding to at least one first state and corresponding stimulation protocols. One or more local field potential signals may be obtained in each first state. At least one second energy value may be determined in each first state. In some embodiments, a period of local field potential signal may be obtained in each first state, to determine a corresponding second energy value. In some embodiments, multiple periods of local field potential signals may be obtained in at least one first state among the multiple first states, and correspondingly, multiple second energy values may be obtained in at least one first state. The method for determining the second energy value may be the same or similar to the method for determining the first energy value described above with reference to FIG. 1, which will not be elaborated again herein.

In some embodiments, the control terminal 510 may be implemented via, for example, a host computer, program control software/program control equipment, etc. In some embodiments, the control terminal 510, when determining the multiple threshold ranges, may be further configured to: determine, in response to receiving one second energy value, two threshold ranges by using the one second energy value as a division point. For example, assuming that the control terminal 510 receives one second energy value a corresponding to one first state, two threshold ranges with a being the dividing point, namely, $(-\infty, a)$, $[a, +(\infty))$, may be determined.

In some embodiments, the control terminal 510, when determining the multiple threshold ranges, may be further configured to: sort, in response to receiving second energy values in the at least one second energy value, the second energy values according to sizes of the second energy values; and determine, by using the sorted second energy values as endpoint values, the plurality of threshold ranges. For example, assuming that the control terminal 510 receives three second energy values a, b, and c corresponding to three first states, and a<b<c, the three second energy values may be sorted in the order of a, b, and c, and the threshold ranges are determined by using a, b, and c as endpoint values. For example, two threshold ranges, i.e., [a, b) and [b, c), may be determined. For another example, four threshold ranges, i.e., $(-\infty, a)$, [a, b), [b, c) and $[c, +(\infty))$, may be determined.

After obtaining the multiple threshold ranges, corresponding stimulation protocols may be configured based on the relative levels between these threshold ranges. The configuration of these stimulation protocols may be implemented either through physician-operated adjustments at the control terminal 510, or via e.g., pre-programmed logic, and machine learning algorithms. The stimulation protocols corresponding to different threshold ranges may be different or the same.

Furthermore, the types of stimulation parameters adjusted across different stimulation protocols may be the same or different, for instance, one stimulation protocol may involve adjusting the amplitude while another may involve adjusting the frequency.

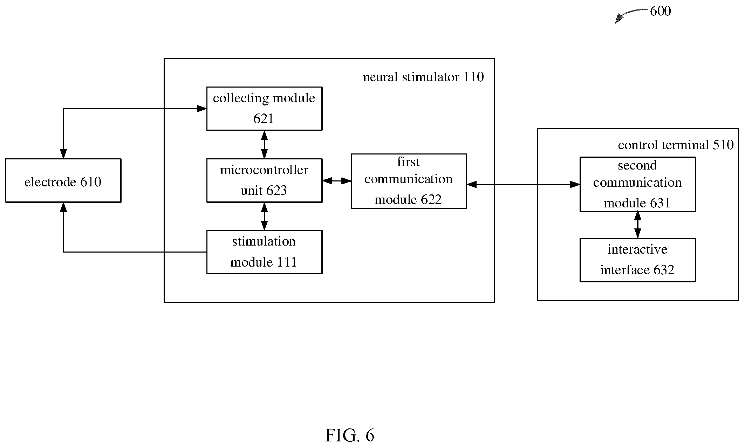

The system including the control terminal according to embodiments of this disclosure and the method for determining multiple threshold ranges are described illustratively above with reference to FIG. 5. As can be appreciated, when the control terminal determines the threshold ranges and corresponding stimulation protocols, and stores the multiple threshold ranges and their associated stimulation protocols in the neural stimulator, this essentially preloads treatment plans developed by physicians for different clinical conditions into the neural stimulator in advance. Consequently, the neural stimulator can perform closed-loop real-time adjustment of stimulation output through continuous monitoring of the patient's condition changes, without requiring hospital visits for manual physician evaluation and adjustment. This approach not only enhances stimulation effects but also provides significant convenience for both physicians and patients, while reducing both the potential disease progression discomfort for patients and the diagnostic-treatment costs for physicians. Furthermore, when the closed-loop stimulation regulation is implemented based on stimulation protocols provided by physicians, it is able to ensure both the accuracy and effects of the stimulation protocols in a better manner. As can be further appreciated, the above description is illustrative rather than limitative. For instance, the system according to the embodiments of this disclosure may include additional components beyond the neural stimulator 110 and the control terminal, such as an electrode. An exemplary description will be provided below with reference to FIG. 6.

FIG. 6 shows a schematic block diagram of a system including an electrode according to the embodiments of this disclosure. As shown in FIG. 6, the system 600 may include the neural stimulator 110, the control terminal 510 and an electrode 610. The electrode 610 may be configured to collect the local field potential signal of the patient's brain region and output a stimulation electrical pulse according to the received stimulation signal. The neural stimulator 110 may include the stimulation module 111, and may further include a collecting module 621, configured to receive the local field potential signal collected by the electrode 610 and send the local field potential signal to the stimulation module 111.

In some embodiments, the electrode 610 may have four contacts, and the four contacts may be used to collect the local field potential signal and output the stimulation electrical pulse. Specifically, in some application scenarios, when using the electrode 610 for collecting, any two of the contacts may be selected as an anode and a cathode, respectively. When using the electrode 610 to output the stimulation electrical pulse, four contacts and a stimulator housing may be set, where the four contacts may be all set as an anode or a cathode, and the housing may be set as the anode. For example, in other application scenarios, monopolar stimulation may be set, that is, the housing serves as an anode, and any one of the contacts serves as a cathode. In addition, bipolar stimulation may also be set, where any two of the contacts serve as an anode and a cathode, respectively. Multi-cathode stimulation may also be set, that is, the housing serves as an anode, and the four contacts serve as a cathode, alternatively, one contact serves as an anode, and the other three contacts serve as a cathode.

In some embodiments, the system according to the embodiments of this disclosure may include an implantable device and an external device (i.e., the control terminal 510). The implantable device may include the neural stimulator 110 and the electrode 610, where the electrode 610 may be implanted in corresponding brain region of the patient's intracranial space, and the neural stimulator 110 may be connected to the electrode 610 and implanted in the patient's skull, so as to deliver the electrical stimulation pulse to the corresponding brain region via the electrode 610. In some embodiments, the electrode 610 may be implanted in a brain region associated with a disease requiring treatment, such as common therapeutic targets for Parkinson's disease including: an internal segment of the globus pallidus (GPi), a subthalamic nucleus (STN), or a ventral intermediate nucleus of the thalamus (Vim). The quantity of electrodes 610 implanted in the patient may be configured as one or multiple in accordance with the practical needs. For example, two electrodes 610 may be implanted to obtain dual-channel electrode data.

In some embodiments, the neural stimulator 110 may further include the collecting module 621, connected to the electrode 610 and configured to control the electrode 610 to collect the local field potential signal of the corresponding brain region, so as to sense the local field potential signal of the patient's brain region in real time through the electrode 610, and send the received local field potential signal to the stimulation module 111 for processing.

As further shown in FIG. 6 , in some embodiments, the neural stimulator 110 may further include: a first communication module 622, which may be configured to send the second energy value; and a microcontroller unit 623, which may be configured to control the stimulation module 111, the first communication module 622 and the collecting module 621, and may be used to merge results from multiple channels, that is, to control the connection and switching of command signals between the processing modules. The control terminal 510 may include: a second communication module 631, which may be connected to the first communication module 622 and may be configured to receive the second energy value, and to send the threshold ranges and corresponding stimulation protocols.

In some embodiments, the first communication module 622 may be wirelessly connected to the second communication module 631. Through the communication link established between the first communication module 622 and the second communication module 631, the collecting instructions, program-controlled stimulation instructions, etc. from the control terminal 510 may also be sent to the neural stimulator 110, and the neural stimulator 110 may also send the real-time obtained LFP signal to the control terminal 510. Furthermore, in some embodiments, the control terminal 510 may also include an interactive interface 632 for human-computer interaction. In some application scenarios, the physician may issue the collecting instructions and/or program-controlled stimulation instructions of the local field potential signal through the interactive interface 632 on the control terminal 510.

With this configuration, the physician can monitor the patient's condition in real time and evaluate the dynamic stimulation effects of the neural stimulator 110. The LFP data can be analyzed to assess disease progression, and to objectively set the threshold ranges and stimulation protocols for optimal therapeutic outcomes based on this. In some embodiments, the system 600 may also include a server, and the control terminal 510 may be connected to the server via 4G, 5G, WIFI, etc., and the LFP data may be uploaded to the server for storage and/or data processing.

The system including the electrode(s), the neural stimulator and the control terminal according to the embodiments of this disclosure is described above with reference to FIG. 6. As can be appreciated, the above description is illustrative rather than limitative. For example, the control terminal 510 is not limited to only including the second communication module and/or the interactive interface, but may also include a processor and a memory, etc. For another example, the system according to the embodiments of this disclosure may not be limited to including the electrode(s), the neural stimulator and the control terminal, but may only include the electrode(s) and the neural stimulator.

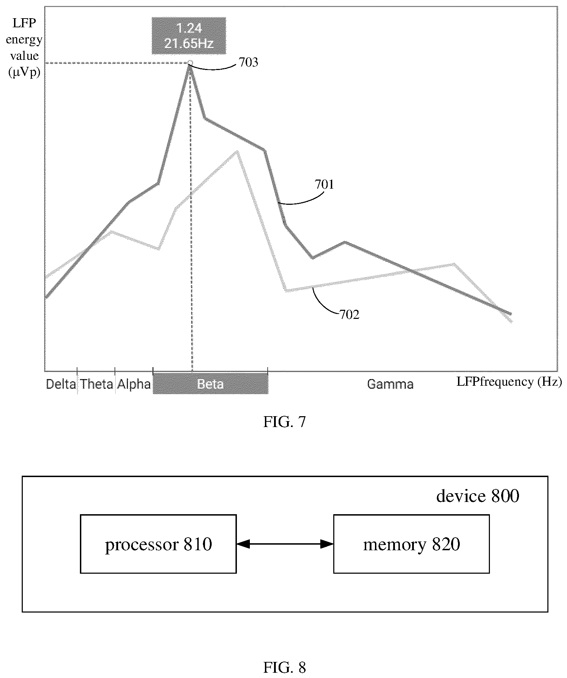

Furthermore, the stimulation module and/or the control terminal may not be limited to performing only the aforementioned operations. For example, in some embodiments, the stimulation module is further configured to: before determining the first energy value, determine, based on local field potential signals of the patient in a plurality of second states, respective third energy values of a plurality of frequency bands in the local field potential signals in the second states, namely, determining the third energy value of each frequency band in each second state; and the control terminal is further configured to: determine, based on a change amount between the third energy values of the frequency bands in the plurality of second states, the frequency band of interest in the plurality of frequency bands. An exemplary description about the method for determining the frequency band of interest will be provided below with reference to FIG. 7.

FIG. 7 shows a line graph of the third energy values of multiple frequency bands in various second states according to the embodiments of this disclosure. As shown in FIG. 7, the horizontal axis of the line graph represents the LFP frequency, which can be categorized into multiple frequency bands according to the magnitude of the frequency, such as the Delta frequency band (0-3 Hz), Theta frequency band (4Hz-7Hz), Alpha frequency band (8Hz-12Hz), Beta frequency band (13Hz-35Hz) and Gamma frequency band (36Hz -200Hz) in the figure. The vertical axis of the line graph represents the third energy value determined based on the LFP signal.

**In** some embodiments, the plurality of second states includes at least one of following: unmedicated with the neural stimulator being not turned on for treatment and medicated with the neural stimulator being not turned on for treatment; or, medicated with the neural stimulator being not turned on for treatment and medicated with the neural stimulator being turned on for treatment. A case where the plurality of second states includes the group of unmedicated with the neural stimulator being not turned on for treatment and medicated with the neural stimulator being not turned on for treatment is taken as an example, the line 701 shown in FIG. 7 is formed by connecting the third energy values of the frequency bands of the local field potential signal detected in the state of unmedicated with the neural stimulator being not turned on for treatment, and the line 702 is formed by connecting the third energy values of the frequency bands of the local field potential signal detected in the state of medicated with the neural stimulator being not turned on for treatment. The method for determining the third energy value may be the same or similar to the method for determining the first energy value described above with reference to FIG. 1, which will not be elaborated again herein.

After receiving the plurality of third energy values in the plurality of second states, the control terminal may perform data processing to form an energy comparison line graph as shown in FIG. 7. This line graph visualizes variations among the third energy values across different second states within each frequency band. In some embodiments, distances between the lines under different second states in the frequency bands may be compared, so that the frequency band with the largest change amount in the third energy value may be determined, and it may be determined as the frequency band of interest.

Taking FIG. 7 as an example, by comparing change amounts between the line 701 and the line 702 across the frequency bands, it can be observed that, in the Beta frequency band (13Hz-35Hz) exhibits the greatest amplitude variation between the line 701 and the line 702, that is, the change amount between the third energy values in the various second states represented by the line 701 and the line 702 is the largest. Notably, a third energy value peak appears at 703 of the line 701, with a frequency of 21.65Hz and a third energy value of $1.24\mu Vp$. Based on this, it is shown that the Beta frequency band is more related to the patient's condition, so the Beta frequency band can be determined as the frequency band of interest.

The method of determining the frequency band of interest according to the embodiments of this disclosure is described above with reference to FIG. 7. As can be appreciated, the above description is illustrative rather than limitative. For example, the manner for determining the change amount between the third energy values of the frequency bands in different second states is not limited to drawing a line graph, the change amounts between the third energy values of the frequency bands in different second states may also be directly calculated, so as to determine the frequency band of interest. For another example, the operation of determining the frequency band of interest may not be limited to being executed by the control terminal, but may also be implemented by the stimulation module.

According to the technical solution of this disclosure, a device for neural stimulation is further provided in the second aspect, which will be described below with reference to Figure 8.

FIG. 8 shows a schematic block diagram of a device for neural stimulation according to the embodiments of this disclosure. As shown in FIG. 8, the device 800 includes a processor 810, configured to execute program instructions; and a memory 820 having program instructions stored thereon, where the program instructions, when loaded and executed by the processor 810, cause the device 800 to perform following operations: determining, based on a received local field potential signal, a first energy value of a frequency band of interest in the local field potential signal; determining a threshold range in which the first energy value is; and outputting, according to a stimulation protocol corresponding to the threshold range, a corresponding stimulation signal.

In some embodiments, the program instructions, when loaded and executed by the processor 810, further cause the device 800 to perform following operations: before determining the threshold range in which the first energy value is, determining, based on a local field potential signal of a patient in a plurality of first states, a second energy value of the frequency band of interest in the local field potential signal in each of the plurality of first states; determining, according to a plurality of second energy values corresponding to at least one first state, a plurality of threshold ranges; and determining, based on the plurality of threshold ranges, respective stimulation protocols.

In some embodiments, the program instructions, when loaded and executed by the processor 810, further cause the device 800 to perform following operations in determining the plurality of threshold ranges: sorting the plurality of second energy values according to sizes of the second energy values; and determining, by using the sorted second energy values as endpoint values, the plurality of threshold ranges.

In some embodiments, the plurality of first states includes at least two of following: unmedicated; medicated; post-medication washout; unmedicated and the neural stimulator is not turned on for treatment; unmedicated and the neural stimulator being turned on for treatment; medicated and the neural stimulator is being turned on for treatment; or, medicated and the neural stimulator being turned on for treatment.

In some embodiments, the program instructions, when loaded and executed by the processor 810, further cause the device 800 to perform following operations: before determining the first energy value, determining, based on local field potential signals of the patient in a plurality of second states, respective third energy values of a plurality of frequency bands in the local field potential signals in the second states; and determining, based on a change amount between the third energy values of the frequency bands in the plurality of second states, the frequency band of interest in the plurality of frequency bands.

In some embodiments, the plurality of second states may include pre-medication and post-medication conditions.

In some embodiments, the program instructions, when loaded and executed by the processor 810, further cause the device 800 to perform following operations in determining the first energy value: determining the first energy value based on following formulas:

$$S_x(k) = \frac{1}{N}|X[k]|^2 \;;\; X[k] = \sum_{n=0}^{N-1} x[n] \times e^{-jkn\omega} = \sum_{n=0}^{N-1} x[n] \times e^{-j2\pi kn/N}$$

;where, $S_x(k)$ represents the energy value, $x[n]$ represents a finite length sequence of length N in the frequency band of interest, $X[k]$ is a discrete Fourier transform pair of the x[n] sequence, $0 \leq k \leq N-1$, N represents the sequence length, a complex exponential signal $e^{jwt} = cos(wt) - jsin(wt)$, an angular frequency of a periodic signal $\omega = 2\pi/N$, e represents a natural constant, and j represents an imaginary unit.

As can be appreciated, the specific implementations of the device according to the embodiments of this disclosure are described above in detail in conjunction with the system implementations, and thus will not be elaborated again herein.

Moreover, in a third aspect of the technical solution of this disclosure, a computer-readable storage medium having computer-readable instructions stored thereon is further provided, where the computer-readable instructions implementing, when executed by one or more processors, the operations performed by any device as described in the second aspect of this disclosure.

The computer-readable storage medium may be any appropriate magnetic storage medium or magneto-optical storage medium, such as Resistive Random Access Memory (RRAM), Dynamic Random Access Memory (DRAM), Static Random Access Memory (SRAM), Enhanced Dynamic Random Access Memory (EDRAM), High-Bandwidth Memory (HBM), Hybrid Memory Cube (HMC), etc., or any other medium that can be used to store the required information and can be accessed by the application, module, or both. Any such computer storage medium may be part of the device or accessible or connectable to the device. Any application or module in this disclosure may be implemented by computer-readable/executable instructions stored or otherwise maintained by the computer-readable medium.

The technical solution for neural stimulation according to this disclosure is described in detail above in combination with multiple embodiments. As can be appreciated, the system according to the embodiments of this disclosure calculates the first energy value of the frequency band of interest in the local field potential signal through the stimulation module, and outputs the corresponding stimulation signal according to the threshold range in which the first energy value is and the corresponding stimulation protocol, so that the system can achieve closed-loop adaptive stimulation control to adapt to the real-time changes in the patient's condition. In addition, by using the induced local field potential (LFP) signal as the basis for treatment and parameter adjustment, it is objective and real-time.

Furthermore, in some embodiments, by comparing the change amounts in the third energy value to determine the frequency band of interest, a targeted plan for individualized diseases can be established, and by identifying the signal frequency bands with higher relevance to different diseases in different patients, a personalized targeted stimulation protocol can be set. As compared with formulating a stimulation protocol based on the full frequency band, when determining the frequency band of interest and determining the threshold range based on the energy value of the frequency band of interest according to the embodiments of this disclosure, it is beneficial to improving the accuracy of stimulation treatment and avoiding blind overstimulation.

Although several embodiments of this disclosure are shown and described herein, it is apparent to a person skilled in the art that such embodiments are for illustrative purposes only. Any modifications, variations, or replacements may occur to the person skilled in the art without departing from the principle and spirit of this disclosure. As can be appreciated, various alternative implementations of the embodiments of this disclosure described herein may be adopted in practicing this disclosure. The appended claims are intended to define the scope of this disclosure and thus cover equivalents or alternatives falling within the scope of these claims.

## Claims

1. A system for neural stimulation, comprising a neural stimulator, wherein the neural stimulator comprises a stimulation module, configured to:

   determine, based on a received local field potential signal, a first energy value of a frequency band of interest in the local field potential signal;
   determine a threshold range in which the first energy value is; and
   output, according to a stimulation protocol corresponding to the threshold range, a corresponding stimulation signal.

2. The system according to claim 1, wherein the stimulation module is further configured to:

   before determining the threshold range in which the first energy value is,
   determine, based on a local field potential signal of a patient in at least one first state, a second energy value of the frequency band of interest in the local field potential signal in each of the at least one first state;
   wherein the system further comprises a control terminal, configured to:

      determine, according to at least one received second energy value corresponding to at least one first state, a plurality of threshold ranges; and
      determine, based on the plurality of threshold ranges, respective stimulation protocols.

3. The system according to claim 2, wherein the control terminal is further configured to:

   sort, in response to receiving second energy values in the at least one second energy value, the second energy values according to sizes of the second energy values; and
   determine, by using the sorted second energy values as endpoint values, the plurality of threshold ranges.

4. The system according to claim 2 or 3, wherein the at least one first state comprises at least one of following:

   unmedicated and the neural stimulator being not turned on for treatment;
   unmedicated and the neural stimulator being turned on for treatment;
   medicated and the neural stimulator being not turned on for treatment; or
   medicated and the neural stimulator being turned on for treatment.

5. The system according to any one of claims 2 to 4, wherein the stimulation module is further configured to:

before determining the first energy value,
determine, based on local field potential signals of the patient in a plurality of second states, respective third energy values of a plurality of frequency bands in the local field potential signals in the second states; and
wherein the control terminal is further configured to:
determine, based on a change amount between the third energy values of the frequency bands in the plurality of second states, the frequency band of interest in the plurality of frequency bands.

6. The system according to claim 5, wherein the plurality of second states comprises at least one of following:

unmedicated with the neural stimulator being not turned on for treatment and medicated with the neural stimulator being not turned on for treatment; or
medicated with the neural stimulator being not turned on for treatment and medicated with the neural stimulator being turned on for treatment.

7. The system according to claim 1, wherein the stimulation module is further configured to:

determine the first energy value based on following formulas:

$$S_x(k) = \frac{1}{N}|X[k]|^2$$

$$X[k] = \sum_{n=0}^{N-1} x[n] \times e^{-jkn\omega} = \sum_{n=0}^{N-1} x[n] \times e^{-j2\pi kn/N}$$

wherein, $S_x(k)$ represents an energy value, $x[n]$ represents a finite length sequence of length N in the frequency band of interest, $X[k]$ is a discrete Fourier transform pair of the x[n] sequence, $0 \leq k \leq N-1$, N represents the sequence length, a complex exponential signal $e^{jwt} = cos(wt) - jsin(wt)$, an angular frequency of a periodic signal $\omega = 2\pi/N$, e represents a natural constant, and j represents an imaginary unit.

8. The system according to any one of claims 1 to 7, further comprising:

an electrode, configured to collect the local field potential signal of the patient's brain region and output a stimulation electrical pulse according to the received stimulation signal; and
wherein the neural stimulator further comprises a collecting module, configured to receive the local field potential signal collected by the electrode and send the local field potential signal to the stimulation module.

9. The system according to claim 2, wherein the neural stimulator further comprises:

a first communication module, configured to send the second energy value; and
a microcontroller unit, configured to control the stimulation module and the first communication module;
wherein the control terminal comprises:
a second communication module, connected to the first communication module and configured to receive the second energy value, and to send the threshold ranges and corresponding stimulation protocols.

10. A device for neural stimulation, comprising:

a processor, configured to execute program instructions; and
a memory having program instructions stored thereon,
wherein the program instructions, when loaded and executed by the processor, cause the device to perform following operations:
determining, based on a received local field potential signal, a first energy value of a frequency band of interest in the local field potential signal;

11. The device according to claim 10, wherein the program instructions, when loaded and executed by the processor, further cause the device to perform following operations:

before determining the threshold range in which the first energy value is,
determining, based on a local field potential signal of a patient in at least one first state, a second energy value of the frequency band of interest in the local field potential signal in each of the at least one first state;
determining, according to at least one second energy value corresponding to at least one first state, a plurality of threshold ranges; and
determining, based on the plurality of threshold ranges, respective stimulation protocols.

**12.** The device according to claim 10, wherein the program instructions, when loaded and executed by the processor, further cause the device to perform following operations in determining the plurality of threshold ranges:

sorting, in response to receiving second energy values in the at least one second energy value, the second energy values according to sizes of the second energy values; and
determining, by using the sorted second energy values as endpoint values, the plurality of threshold ranges.

**13.** The device according to claim 11 or 12, wherein the at least one first state comprises at least one of following:

unmedicated and the neural stimulator being not turned on for treatment;
unmedicated and the neural stimulator being turned on for treatment;
medicated and the neural stimulator being not turned on for treatment; or
medicated and the neural stimulator being turned on for treatment.

**14.** The device according to any one of claims 11 to 13, wherein the program instructions, when loaded and executed by the processor, further cause the device to perform following operations:

before determining the first energy value,
determining, based on local field potential signals of the patient in a plurality of second states, respective third energy values of a plurality of frequency bands in the local field potential signals in the second states; and
determining, based on a change amount between the third energy values of the frequency bands in the plurality of second states, the frequency band of interest in the plurality of frequency bands.

**15.** The device according to claim 14, wherein the plurality of second states comprises at least one of following:

unmedicated with the neural stimulator being not turned on for treatment and medicated with the neural stimulator being not turned on for treatment; or
medicated with the neural stimulator being not turned on for treatment and medicated with the neural stimulator being turned on for treatment.

**16.** The device according to claim 10, wherein the program instructions, when loaded and executed by the processor, further cause the device to perform following operations in determining the first energy value:

determining the first energy value based on following formulas:

$$S_x(k) = \frac{1}{N}|X[k]|^2$$

$$X[k] = \sum_{n=0}^{N-1} x[n] \times e^{-jkn\omega} = \sum_{n=0}^{N-1} x[n] \times e^{-j2\pi kn/N}$$

wherein, $S_x(k)$ represents the energy value, $x[n]$ represents a finite length sequence of length N in the frequency band of interest, $X[k]$ is a discrete Fourier transform pair of the $x[n]$ sequence, $0 \le k \le N-1$, N represents the sequence length, a complex exponential signal $e^{jwt} = cos(wt) - jsin(wt)$, an angular frequency of a periodic signal $\omega=2\pi[/N$, e represents a natural constant, and j represents an imaginary unit.

**17.** A computer readable storage medium having computer-readable instructions stored thereon, the computer-readable instructions implementing, when executed by one or more processors, the operations performed by the device according to any one of claims 10 to 16.

100

neural stimulator 110

stimulation module
111

FIG. 1

receive a local field potential signal of a brain region in real time — 210

determine the first energy value n1 of the frequency band of interest in the local field potential signal — 220

determine the threshold range in which the first energy value n1 is — 230

NO ← n1<a → YES → 241 stimulation protocol A

NO ← a≤n1<b → YES → stimulation protocol B — 242

b≤n1<c → YES → stimulation protocol C → NO — 243

c≤n1 → YES → stimulation protocol D — 244 → NO

FIG. 2

FIG. 3

FIG. 4

FIG. 5

FIG. 6

FIG. 7

FIG. 8

## INTERNATIONAL SEARCH REPORT

| International application No. |
|---|
| **PCT/CN2023/139589** |

**A.  CLASSIFICATION OF SUBJECT MATTER**

A61N1/36(2006.01)i

According to International Patent Classification (IPC) or to both national classification and IPC

**B.  FIELDS SEARCHED**

Minimum documentation searched (classification system followed by classification symbols)

IPC:A61N1

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI: 神经, 刺激, 频率, 频段, 能量值, 阈值, 范围, 区间, neuro, stimulation, frequency, energy, power, threshold, range

**C.  DOCUMENTS CONSIDERED TO BE RELEVANT**

| Category* | Citation of document, with indication, where appropriate, of the relevant passages | Relevant to claim No. |
|---|---|---|
| PX | CN 115814271 A (HANGZHOU NUOWEI MEDICAL TECHNOLOGY CO., LTD.) 21 March 2023 (2023-03-21)<br>entire document | 1-17 |
| Y | US 2021402172 A1 (CALA HEALTH, INC.) 30 December 2021 (2021-12-30)<br>description, paragraphs [0064]-[0115], and figures 1-6 | 1-17 |
| Y | US 2021128920 A1 (DUKE UNIVERSITY) 06 May 2021 (2021-05-06)<br>description, paragraphs [0051]-[0129], and figures 1-14 | 1-17 |
| A | CN 113426015 A (BEIJING BRAINUP TECHNOLOGY CO., LTD.) 24 September 2021 (2021-09-24)<br>entire document | 1-17 |
| A | US 2011040353 A1 (MEDTRONIC, INC.) 17 February 2011 (2011-02-17)<br>entire document | 1-17 |

☐ Further documents are listed in the continuation of Box C.   ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **14 March 2024** | **18 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/139589**

| Patent document cited in search report | | | Publication date (day/month/year) | Patent family member(s) | | | Publication date (day/month/year) |
|---|---|---|---|---|---|---|---|
| CN | 115814271 | A | 21 March 2023 | None | | | |
| US | 2021402172 | A1 | 30 December 2021 | EP | 3856330 | A1 | 04 August 2021 |
| | | | | EP | 3856330 | A4 | 15 June 2022 |
| | | | | EP | 3856330 | B1 | 10 January 2024 |
| | | | | WO | 2020069219 | A1 | 02 April 2020 |
| | | | | CN | 113164744 | A | 23 July 2021 |
| US | 2021128920 | A1 | 06 May 2021 | US | 11351378 | B2 | 07 June 2022 |
| | | | | WO | 2018119220 | A1 | 28 June 2018 |
| | | | | US | 2022323765 | A1 | 13 October 2022 |
| | | | | EP | 4223358 | A1 | 09 August 2023 |
| | | | | EP | 3558445 | A1 | 30 October 2019 |
| | | | | EP | 3558445 | A4 | 22 July 2020 |
| | | | | EP | 3558445 | B1 | 07 June 2023 |
| CN | 113426015 | A | 24 September 2021 | None | | | |
| US | 2011040353 | A1 | 17 February 2011 | US | 2014031900 | A1 | 30 January 2014 |
| | | | | US | 8805525 | B2 | 12 August 2014 |
| | | | | US | 8548596 | B2 | 01 October 2013 |
| | | | | WO | 2009134480 | A1 | 05 November 2009 |

Form PCT/ISA/210 (patent family annex) (July 2022)